# EUROPEAN PATENT SPECIFICATION

(11) **EP 2 168 608 B1**
(45) Date of publication and mention of the grant of the patent: **28.01.2015**
(21) Application number: 09171452.7
(22) Date of filing: 28.09.2009
(51) Int. Cl.: A61L 15/58, C09J 7/04

(54) **Pressure-sensitive adhesive tape or sheet for application to skin, and process for producing the same**
Haftklebetape oder -folie zur Anwendung auf die Haut, und Herstellungsverfahren dafür
Bande ou feuille adhésive sensible à la pression à appliquer sur la peau, et son procédé de production

(30) Priority: 30.09.2008 JP 2008253232
(43) Date of publication of application: 31.03.2010
(73) Proprietor: NITTO DENKO CORPORATION, Ibaraki-shi, Osaka 567-8680 (JP)
(72) Inventor: Takahashi, Hisanori, Ibaraki-shi Osaka 567-8680 (JP); Kamata, Kouji, Ibaraki-shi Osaka 567-8680 (JP); Oohira, Osamu, Osaki-shi Miyagi (JP); Yoshikawa, Toshiyuki, Ibaraki-shi Osaka 567-8680 (JP)
(74) Representative: Grünecker, Kinkeldey, Stockmair & Schwanhäusser

(56) References cited:
- EP-A2- 1 184 039
- DE-A1- 19 925 972
- JP-A- 2003 313 110
- JP-A- 2004 049 541
- US-A1- 2001 028 943
- TRI-STATE HOSPITAL SUPPLY CORPORATION, MANUFACTURERS OF CENTURION HEALTHCARE PRODUCTS: 'VersaDerm 2000 Fabric Border Dressing, Technical Monograph', [Online] 13 March 2006, pages 1 - 4 Howell, USA Retrieved from the Internet: <URL:http://web.archive.org/web/20060313071 235/http://www.tshsc.com/images/literature_ pdfs/versadermtech.pdf> [retrieved on 2010-09-22]

## Description

### FIELD OF THE INVENTION

The present invention relates to a pressure-sensitive adhesive tape or sheet for application to skin, which is used in various fields of medicine, sports, or the like.

### BACKGROUND OF THE INVENTION

Heretofore in the fields of medicine and sports, pressure-sensitive adhesive tapes or sheets for application to skin (hereinafter may also be referred as "pressure-sensitive adhesive tapes") are used in various applications such as first-aid adhesive tapes, plasters, taping adhesive tapes, and the like; and recently, various pressure-sensitive adhesive tapes manufactured into various forms depending on the use thereof have been produced and soid.

Herein, taping means fixing the treated part such as joint part or muscle with pressure-sensitive adhesive tapes. It is effective for relieving pain, promoting the curing of the affected part or the like by supporting, reinforcing or pressing the part with the tape. Such a taping therapy is a means effective for relieving pain relatively inexpensively and readily, as compared with other therapeutical methods such as drug therapy, hospital visit therapy or surgery, and it also shows a significant effect on prior prevention and for ex-post emergency treatment of sports injury.

The pressure-sensitive adhesive tape for use in taping is, in fact, required to have various functions and properties such as elasticity, elastic memory characteristics (restorability or pressure characteristics), low stimulation, low modulus, flexibility and water repellency, in addition to the original, pressure-sensitive adhesive function of the tape applicable to skin. For example, the surface of the skin to which, in general, a pressure-sensitive adhesive tape is attached is a roughened surface with dead skin cells (horn), sweat, sebum, dust and the like adhering thereto, and is a surface that expands and contracts with body movement. In particular, the pressure-sensitive adhesive tape to be attached to the part that bends and moves greatly such as joint part is required to be able to follow the flexing movement of the skin. Furthermore, in taping, the pressure-sensitive adhesive tape is in general attached to the skin so that it tightens the skin surface, and therefore the pressure-sensitive adhesive tape is also required to have an elastic characteristic by itself in some degree as one important characteristic thereof.

However, in case where such a pressure-sensitive adhesive tape having an elastic characteristic is used, its force of tightening (tightening force) may reduce and the pressure-sensitive adhesive tape may loosen with the lapse of time after its attaching to skin in some cases, Accordingly, the pressure-sensitive adhesive tape is required to additionally have a suitable elastic memory characteristic. In particular, in the case of taping a part of which range of motion may be great or moving intensely while taped, the elastic memory characteristic of the tape is an important characteristic for the purpose of sustaining the fixation of the taped part (see JP-A-2004-49541).

Furthermore, when a pressure-sensitive adhesive having such a skin adhesive force that may excessively restrict the movement of skin or a supporting substrate that may excessively restrict the movement of skin is used in the pressure-sensitive adhesive tape, there may occur some physical stress between the pressure-sensitive adhesive tape and the skin taped with it, and as a result, the tape may stimulate the taped part of the skin to cause skin irritation, thereby giving uncomfortable feeling and unpleasant sensation to the user. Accordingly, in the pressure-sensitive adhesive tape that must be applied to a movable part such as joint part, it is desirable to use a supporting substrate that is elastic in a degree capable of suitably following the movement of a skin surface and can readily expand and contract under small stress so as not to restrict the skin surface on which it has expanded, or that is, a supporting substrate having a low modulus.

In addition, the pressure-sensitive adhesive layer in such a pressure-sensitive adhesive tape for use for taping is further required to have flexibility and viscoelasticity enough for elastic deformation in accordance with the movement of skin, capable of following and firmly attaching to a skin surface soiled with sweat and dirt and to a roughened skin surface. Accordingly, the pressure-sensitive adhesive for the layer is preferably such that it is adhesive to skin to such a degree that it does not peel away from the skin surface while it is kept attached thereto, but when the tape is taken off from the skin, it may be readily peeled away therefrom at a weak peeling force not causing skin irritation. In tape peeling, when the skin surface is not damaged by dead-skin removal or the like, then the tape may not give any excessive and superfluous irritation to the skin surface to which it has attached, and therefore the skin trouble of irritation or the like can be minimized.

Further, in taping with a pressure-sensitive adhesive tape, the tape is tightly attached to the skin surface of the part to be treated and is elastically wound around the part under tension, and for this, it is preferred that the supporting substrate of the pressure-sensitive adhesive tape does not fray.

On the other hand, as a supporting substrate for the pressure-sensitive adhesive tape or sheet for application to skin for use for taping or the like, heretofore used are fabrics such as elastic woven fabrics produced by interweaving elastic yarn in cotton woven fabrics, or hard twist fabrics formed of twist yarn etc. In general, the pressure-sensitive adhesive tape including such a woven fabric has a low modulus and has a suitable mechanical strength, but is, on the contrary, poorly elastic and has poor elastic memory characteristic, and therefore has a problem in that its fixation to the treated part is poor and the attached tape may often slip off.

In addition, in case where the pressure-sensitive adhesive tape employing the supporting substrate of the type is not repellent to water and when the user taped with it moves and sweats in large quantities or when the user is wetted with water at the time of rainy weather, then the supporting substrate may be kept watered; and in such a case where the pressure-sensitive adhesive tape is wound around the joint part of the user, the pressure-sensitive adhesive force of the pressure-sensitive adhesive tape in the area between the back side thereof and the pressure-sensitive adhesive layer side thereof may lower, therefore causing a problem in that the tape may often peel away.

Accordingly, as the substrate for the pressure-sensitive adhesive tape, a film, a non-woven fabric or the like formed of a polyurethane resin or a polyolefin resin has been also used. Such a substrate has a low modulus, but is poorly elastic and has a low mechanical strength, and is therefore problematic in that the pressure-sensitive adhesive tape employing the substrate is difficult to handle when applied to skin. Recently, a pressure-sensitive adhesive tape employing a non-woven fabric of an elastic polyurethane material has been developed, and this has solved a problem of elasticity and fittability of the tape to the part to be treated. However, the problem of poor mechanical strength of the tape has not been solved yet. In addition, products produced by laminating polyurethane film on a fabric also been developed. At present, however, a pressure-sensitive adhesive tape having good mechanical strength and elastic memory characteristic enough for application to intensely moving parts such as legs and knees and having sufficient fixability to the applied parts have not been obtained yet (see JP-A-2004-49541 ).

EP 1 184 039 A2 discloses a process for producing an adhesive tape comprising the steps of preparing an adhesive composition by mixing an acrylic copolymer, triglyceryl caprylate and a crosslinking agent; applying the adhesive composition to a silicone-treated release sheet; drying the composition to form an adhesive layer; and pressing a polyester nonwoven fabric to the surface of the obtained adhesive layer. Different water repellent treatments of substrates are disclosed in DE 199 25 972 A1, US 2001/028943 A1 and JP 2003-313110 A.

### SUMMARY OF THE INVENTION

Taking the above-mentioned current situation into consideration, the present invention provides a pressure-sensitive adhesive tape or sheet for application to skin, which can be firmly attached to skin and has good fixability thereto without loosening while used, and good followability to the movement of the skin surface and which can minimize the irritation to skin.

Namely, the present invention provides the following items (1) to (10).
(1) A pressure-sensitive adhesive tape or sheet for application to skin, which comprises:
   a stretch fabric being a knitted fabric, and
   a pressure-sensitive adhesive layer provided on one surface of the stretch fabric;
   wherein the pressure-sensitive adhesive layer comprises:
   an acrylic copolymer obtained from a monomer mixture comprising an alkyl (meth)acrylate of from 40 to 80% by weight, an alkoxy (meth)acrylate of from 10 to 50% by weight and a (meth)acrylic acid of from 1 to 10% by weight,
   a long-chain fatty acid glycerin ester in an amount of from 20 to 100 parts by weight relative to 100 parts by weight of the acrylic copolymer, and
   a crosslinking agent,
   said pressure-sensitive adhesive layer being crosslinked with the crosslinking agent,
   wherein a water-repellant film is not laminated on the fabric, and
   wherein the stretch fabric is subjected to a water-repellent treatment with a water repellent.
(2) The pressure-sensitive adhesive tape or sheet according to (1) above, wherein the water repellent is an emulsion containing an acrylic water repellent containing a perfluoroalkyl (meth)acrylate as a main ingredient thereof.
(3) The pressure-sensitive adhesive tape or sheet according to (1) above, wherein the water-repellent treatment comprises dipping the stretch fabric in an emulsion containing the water repellent wringing and drying the stretch fabric to thereby make an acrylic water repellent contained in the water repellent adhere to surfaces of fibers constituting the stretch fabric.
(4) The pressure-sensitive adhesive tape or sheet according to (1) above, wherein a fraction of the acrylic copolymer soluble in a solvent for molecular weight determination has a weight-average molecular weight of from 500,000 to 1,000,000 and has a molecular weight distribution of from 8 to 15, wherein the weight-average molecular weight and the molecular weight distribution are data determined through gel permeation chromatography, the sample to be analyzed is dissolved in tetrahydrofuran, then the soluble fraction thereof having passed through a membrane filter having a diameter of 0.45 µm is analyzed, and the data are computed in terms of polystyrene.
(5) The pressure-sensitive adhesive tape or sheet according to (1) above, wherein the acrylic copolymer has a molecular weight distribution of from 3 to 22.
(6) The pressure-sensitive adhesive tape or sheet according to (1) above, wherein the stretch fabric has a degree of elongation of at least 30% in both the machine direction and the cross direction thereof.
(7) A process for producing a pressure-sensitive adhesive tape or sheet, said process comprising:
   applying a pressure-sensitive adhesive solution comprising an acrylic copolymer obtained from a monomer mixture comprising an alkyl (methacrylate of from 40 to 80% by weight, an alkoxy (meth)acrylate of from 10 to 50% by weight and a (meth)acrylic acid of from 1 to 10% by weight, a long-chain fatty acid glycerin ester in an amount of from 20 to 100 parts by weight relative to 100 parts by weight of the acrylic copolymer and a crosslinking agent, onto one surface of a release liner to a predetermined thickness to thereby form a pressure-sensitive adhesive solution layer thereon;
   then attaching the pressure-sensitive adhesive solution layer on one surface of a stretch fabric being a knitted fabric while the pressure-sensitive adhesive solution layer is not completely dried: and
   then drying the pressure-sensitive adhesive solution layer to form a pressure-sensitive adhesive layer, followed by crosslinking the pressure-sensitive adhesive layer,
   wherein the stretch fabric is previously subjected to a water-repellent treatment with a water repellent.
(8) The process according to (7) above, wherein the water repellent is an emulsion containing an acrylic water repellent containing a perfluoroalkyl (meth)acrylate as a main ingredient thereof.
(9) The process according to (7) above, wherein the water-repellent treatment comprises dipping the stretch fabric in an emulsion containing the water repellent, wringing and drying the stretch fabric to thereby make an acrylic water repellent contained in the water repellent adhere to surfaces of fibers constituting the stretch fabric.
(10) The process according to (7) above, wherein the stretch fabric has a degree of elongation of at least 30% in both the machine direction and the cross direction thereof.

As described in the above, since the pressure-sensitive adhesive tape or sheet for application to skin of the present invention includes a pressure-sensitive adhesive layer formed on one surface of a stretch fabric, in which the layer contains an acrylic copolymer and a long-chain fatty acid glycerin ester and is crosslinked; it is elastic, as capable of following the movement of the skin surface to which is it attached, and causes little skin irritation. Accordingly, especially when used for taping, it enables long-term stable fixation, and even when used in a part that may move greatly or even when the part taped with it is intensely moved, it does not peel off, and is therefore most suitable for a pressure-sensitive adhesive tape or sheet for taping.

In particular, when the pressure-sensitive adhesive tape is subjected to a certain water-repellent treatment, the whole tape can be excellently repellent to water, and therefore, even when wetted with water, the tape hardly peels off and its adhesive force to skin hardly lowers as resistant to penetration thereinto of external water such as rainwater.

According to the production process of the present invention, since a pressure-sensitive adhesive solution layer is, not as yet completely dried, or that is, while semidried, laminated on one surface of a stretch fabric, the pressure-sensitive adhesive layer may be firmly anchored to the stretch fabric. In this case, when a water repellent firmly adheres to the surfaces of the fibers constituting the stretch fabric in the water-repellent treatment of the stretch fabric, it brings about another advantage in that the pressure-sensitive adhesive layer laminated on the stretch fabric, while semidried, does neither detract from the moisture permeability that the stretch fabric originally has, nor penetrate through the stretch fabric subjected to the water-repellent treatment, to the back thereof (back-through-penetration).

### DETAILED OF THE INVENTION

The stretch fabric of the pressure-sensitive adhesive tape or sheet for application to skin of the present invention is, for example, one that is elastic enough for use in taping application; and specifically used is a fabric having a degree of elongation of at least 30%, preferably at least 50% in both the machine direction and the cross direction thereof. In particular, for use to the joint part that may bend greatly such as elbows and knees, preferred is a fabric having a degree of elongation of about from 100% to 130%.

The fabric used is a knitted fabric for exhibiting good flexibility. As the knitted fabric, preferred is one having a thickness of about from 0.2 to 1.0 mm, more preferably from about 0.4 to 0.8 mm, since the knitted fabric of the type has a suitable mechanical strength and does not cause any unpleasant feeling when applied to skin.

As the material of the fibers constituting the stretch fabric, usable are natural fibers of cotton or the like and various plastic fibers. From the viewpoint of the fiber strength in stretch operation, employable are knitted fabrics of high-stretch fibers such as stretch yarns produced by specifically processing polyamide (nylon) or polyester fibers for making them stretchable, as well as polyurethane elastic yarns, and woven fabrics of non-stretch fibers and stretch fibers. Preferred are high-stretch knitted tricot fabrics containing from 80 to 95% by weight of fiber yarns of polyamide, polyester, cotton or the like and from 5 to 20% by weight of polyurethane elastic yarns. Regarding the knitting mode for the fabrics, herein employable is any of warp knitting mode including tricot knitting, Raschel knitting and Milanese knitting, or weft knitting mode including plain knitting and circular knitting. More preferred is warp knitting, as the fabric hardly frays when cut.

In particular, as the knitted stretch fabric, preferred is one produced by knitting stretch yarns of polyurethane or the like in a spun-bond non-woven fabric, as well as a conjugate woven fabric produced by weaving stretch yarns and non-stretch yarns of polyester or the like. For example, preferred for use herein is a stretch knitted fabric produced by knitting a polyester stretch yarn "Lycra" (trade name, manufactured by Du Pont-Toray Co., Ltd.) and a polyester yarn in a polyester spun-bond non-woven fabric "Ecule 6201A" (trade name, manufactured by TOYOBO CO., LTD.).

Not specifically limited, the stretch yarn may be any one that is elastic and stretchable. Especially preferred is a polyurethane elastic yarn, and more preferred is the yarn having a fiber diameter of about from 40 to 160 deniers, as preferable for making the tape having the stretchability enough for use for taping applications.

The stretch fabric for use in the present invention is subjected to a water-repellent treatment. Specifically, a water-repellent film such as a polyurethane film is not laminated on a fabric, but instead it is preferable that the surfaces of the fibers constituting the fabric are coated with a water repellent. This is because, in case where a film or the like is laminated on a fabric so as to make the pressure-sensitive adhesive tape repellent to water by itself, then it may detract from the excellent vapor permeability that the fabric originally has, even when a film having a high vapor permeability is used; and in such a case, when the tape is kept attached to skin for a long period of time, then the skin may get sweaty and may be irritated. On the other hand, in case where the surface of every fiber constituting the fabric is subjected to a water-repellent treatment, then the pressure-sensitive adhesive tape may be made repellent to water by itself, without detracting from the vapor permeability of the fabric itself.

The water repellent to be used for the water-repellent treatment is not specifically limited. From the viewpoint of the easy handlability and the excellent function of water repellency, preferred is a water repellent containing a fluororesin as the main ingredient thereof; and from the viewpoint of the excellent oil-repellent and water-repellent characteristics and the durability, preferred is an emulsion containing an acrylic water repellent containing a perfluoroalkyl (meth)acrylate as the main ingredient thereof. Since the perfluoroalkyl (meth)acrylate-based water repellent has both water-repellent and oil-repellent characteristics, when it is applied to the stretch fabric for use in the present invention, then the fabric can be repellent not only to water but also to oil such as the pressure-sensitive adhesive and also the solvent which dissolves the pressure-sensitive adhesive.

The water-repellent treatment with the above-mentioned water repellent may be attained by spraying the stretch fabric with the water repellent or by dipping it in the water repellent. Preferred is the dipping treatment for the purpose of surely and uniformly adhering the water repellent onto the surfaces of the fibers constituting the fabric. Specifically, the stretch fabric is dipped in an emulsion containing the water repellent to thereby make the surfaces of the fibers well wetted with the water repellent, then wringed with a pinch roll or the like to thereby remove the excessive water repellent therefrom, and thereafter dried with natural air or with hot air to attain the water-repellent treatment.

As a result of the water-repellent treatment of the stretch fabric in the manner as above, the pressure-sensitive adhesive tape or sheet for application to skin of the present invention can be protected from being wetted with external water, and in addition, even when the taped part is moved by exercise and becomes sweaty, the pressure-sensitive adhesive force of the pressure-sensitive adhesive layer of the tape to the skin is hardly lowered and therefore the tape can keep excellent taping performance.

In the present invention, the pressure-sensitive adhesive layer is formed on one surface of the stretch fabric. The pressure-sensitive adhesive layer is formed of a pressure-sensitive adhesive containing an acrylic copolymer as the main ingredient thereof, because the pressure-sensitive adhesive force thereof can be easily controlled, it causes little skin irritation and so on. Above all, a gel-type adhesive of little skin irritation is used. Specifically, herein used is an acrylic copolymer obtained from a monomer mixture containing an alkyl (meth)acrylate of from 40 to 80% by weight, an alkoxy (meth)acrylate of from 10 to 50% by weight and a (meth)acrylic acid of from 1 to 10% by weight. In addition, the pressure-sensitive adhesive for use in the present invention further contains a long-chain fatty acid glycerin ester incorporated therein in an amount of from 20 to 100 parts by weight relative to 100 parts by weight of the acrylic copolymer, and also contains a crosslinking agent, and the pressure-sensitive adhesive layer is crosslinked with the crosslinking agent as a whole.

The alkyl (meth)accylalte in the acrylic copolymer is a main ingredient for making the pressure-sensitive adhesive layer sticky and adhesive to skin, and is effective when the alkyl group therein is a long-chain alkyl group having at least 6 carbon atoms, more preferably from 6 to 18 carbon atoms. The alkyl (meth)acrylate is advantageous in that its property to irritate skin is relatively low and its adhesive force hardly lowers even in long-term use.

Specific examples of the alkyl (meth)acrylate include butyl ester, propyl ester, octyl ester, nonyl ester, decyl ester, dodecyl ester and lauryl ester of acrylic acid or methacrylic acid; and one or more of these may be used herein either singly or as combined. Needless-to-say, the alkyl ester chain may be linear or branched.

The alkyl (meth)acrylate is copolymerized with the alkoxy (meth)acrylate and (meth)acrylic acid to be mentioned below, thereby giving an acrylic copolymer having adhesiveness. In the present invention, the alkyl (meth)acrylate is copolymerized within a range of from 40 to 80% by weight, preferably from 50 to 75% by weight. When the level of copolymerization with the alkyl (meth)acrylate is less than 40% by weight, then the produced copolymer could not exhibit sufficient skin adhesiveness; while it is more than 80% by weight, then the cohesive force of the copolymer may be low, and when the tape is peeled away from the surface of a skin, there may occur a phenomenon of adhesive residue on the skin.

The alkoxy (meth)acrylate to be copolymerized with the above-mentioned alkyl (meth)acrylate is an ingredient that makes the resulting copolymer have water vapor permeability, or that is, moisture permeability. Accordingly, it is desirable that the ingredient is copolymerized in an amount of from 10 to 50% by weight, preferably from 20 to 45% by weight of the acrylic copolymer. Specifically, preferred for use herein is an alkoxyalkyl acrylate where the alkoxy group has from 1 to 4 carbon atoms, such as methoxypolyethylene glycol acrylate, ethoxydiethylene glycol acrylate, butoxydiethylene glycol acrylate, methoxyethyl acrylate, ethoxyethyl acrylate or butyoxyethyl acrylate.

In the present invention, (meth)acrylic acid, or that is, acrylic acid and/or methacrylic acid is copolymerized along with the above-mentioned alkoxy (meth)acrylate. Copolymerization with (meth)acrylic acid enhances the cohesive force of the resulting acrylic copolymer, and therefore this is an extremely important monomer in preparing the pressure-sensitive adhesive. However, when a large quantity of (meth)acrylic acid is copolymerized, then the skin irritation with the resulting copolymer adhesive may increase though the cohesive force thereof may be expected to increase. Therefore, in the present invention, the copolymerization ratio of the ingredient is preferably about from 1 to 10% by weight, more preferably about from 3 to 7% by weight.

The acrylic copolymer to be incorporated in the pressure-sensitive adhesive for use in the present invention is a copolymer containing the above-mentioned monomers as the indispensable ingredients, and if desired, the copolymer may be further copolymerized with an additional modifying monomer such as styrene, vinyl acetate or N-vinyl-2-pyrrolidone for modifying the copolymer in various way, for example, for making the copolymer hydrophilic.

In the pressure-sensitive adhesive layer in the present invention, it is important that a long-chain fatty acid glycerin ester is incorporated into the above-mentioned acrylic copolymer. This ingredient is necessary for making the pressure-sensitive adhesive layer flexible to thereby reduce the skin irritation with the layer, and it is incorporated in an amount of about from 20 to 100 parts by weight, preferably about from 25 to 80 parts by weight relative to 100 parts by weight of the acrylic copolymer. Examples of the long-chain fatty acid glycerin ester include monoglyceryl caprylate, triglyceryl caprylate, triglyceryl 2-ethylhexanoate, triglyceryl caprate, triglycerin laurate, triglyceryl isostearate and triglyceryl trioleate. Of these, preferred is a saturated fatty acid having no unsaturated double bond from the viewpoint of antioxidation, and also preferred is a glycerin ester of such a saturated fatty acid from the viewpoint of low skin irritation. Accordingly, preferred is a glycerin ester of a saturated fatty acid such as capryHc acid, capric acid and 2-ethylhexanoic acid. Specifically preferred is triglyceryl caprylate, triglyceryl caprare and triglyceryl 2-ethylhexanoate. From the viewpoint of the miscibility thereof with the acrylic copolymer, more preferred is triglyceryl caprylate.

In the present invention, the pressure-sensitive adhesive layer must be crosslinked in order that the layer can maintain a suitable level of cohesive force. This is because the mere addition of the above-mentioned long-chain fatty acid glycerin ester to the pressure-sensitive adhesive layer is insufficient since the cohesive force of the layer becomes low and thus there may occur a phenomenon of adhesive residue in application of the pressure-sensitive adhesive tape to skin and in removal thereof from the skin. Crosslinking 17 treatment includes physical crosslinking by irradiation of ionizing radiation, such as electron beams, γ rays, X rays or the like, and chemical crosslinking with a crosslinking agent. In the present invention, preferred is chemical crosslinking treatment with a crosslinking agent from the viewpoint of the handlability and the reproducibility in the treatment. Specifically, a crosslinking agent is added to the pressure-sensitive adhesive composition for constituting the pressure-sensitive adhesive layer, which contains the acrylic copolymer, the long-chain fatty acid glycerin ester and any other optional ingredient, and the composition is then heated and crosslinked. The crosslinking agent may be any ordinary one for use for crosslinking the pressure-sensitive adhesive, such as an isocyanate-based crosslinking agent, a peroxide-based crosslinking agent and a metal chelate-based crosslinking agent.

The pressure-sensitive adhesive layer thus crosslinked in the manner as above may have well-balanced properties of skin adhesiveness and cohesiveness, depending on the degree of crosslinking thereof. However, the molecular weight and the molecular weight distribution of the acrylic copolymer may also have some influence on controlling the properties. Specifically, when the molecular weight of the acrylic copolymer is too large, then the flexibility of the pressure-sensitive adhesive layer may tend to lower; while when it is too small, then the cohesive force thereof may tend to lower. In addition, it is empirically known that the copolymer having a suitable molecular weight distribution may have well-ballanced adhesive characteristics. As a result, in the present invention, the acrylic copolymer is preferably controlled such that the weight-average molecular weight of the fraction thereof soluble in a solvent for molecular weight determination may fall within a range of about from 500,000 to 1,000,000, more preferably about from 600,000 to 900,000, the number-average molecular weight of the faction thereof soluble in a solvent for molecular weight determination may fall within a range of from 30,000 to 190,000, and the molecular weight distribution of the copolymer may fall within a range of about from 3 to 22, more preferably about from 8 to 15.

Herein, the weight-average molecular weight and the molecular weight distribution are the data determined through gel permeation chromatography (GPC). The sample to be analyzed is dissolved in tetrahydrofuran, then the soluble fraction thereof having passed through a membrane filter having a diameter of 0.45 µm is analyzed, and the data no computed in terms of polystyrene.

The thickness of the pressure-sensitive adhesive layer having the above-mentioned constitution is preferably about from 20 to 150 µm, more preferably about from 30 to 90 µmn from the viewpoint that it may exhibit a suitable adhesiveness to skin in taping applications, or the like. From the viewpoint of satisfying both the fixation on skin and the low skin irritation, the characteristics of the pressure-sensitive adhesive layer are preferably designed as follow. Namely, the pressure-sensitive adhesive force of the layer, or that is, the pressure-sensitive adhesive force thereof to a Bakelite board is at most about 10 N/19 mm width or less, preferably about from 0.8 to 5 N/19 mm width, as measured according to a 180-degree peeling method of JIS. The thus-designed pressure-sensitive adhesive layer may have good adhesiveness to skin.

The pressure-sensitive adhesive tape or sheet for application to skin of the present invention has the above-mentioned constitution. For the purpose of protecting the surface of the pressure-sensitive adhesive layer thereof from being fouled, it is preferred that the surface of the pressure-sensitive adhesive layer is coated with a release liner until use. The release liner may be any one generally used in pressure-sensitive adhesive tapes for application to skin. Specifically, examples thereof include those produced by coating the surface of high-quality paper, glassine paper, parchment paper or the like with a release agent such as a silicone resinor a fluororesin; and those produced by coating the surface of high-quality paper anchor-coated with resin or laminated with polyethylene, with a release agent such as a silicone resin or a fluororesin.

One embodiment of a production process for the pressure-sensitive adhesive tape or sheet for application to skin of the present invention is described below, to which, however, the present invention should not be limited.

As the pressure-sensitive adhesive for constituting the pressure-sensitive adhesive layer as above, an acrylic copolymer solution is prepared by copolymerization in a mode of ordinary radical polymerization in an organic solvent of a monomer mixture containing an alkyl (meth)acrylate of from 40 to 80% by weight, an alkoxy (meth)acrylate of from 10 to 50% by weight and a (meth)acrylic add of from 1 to 10% by weight. Next, a long-chain fatty acid glycerin ester is added thereto in an amount of from 20 to 100 parts by weight relative to 100 parts by weight of the solid fraction (acrylic copolymer fraction) of the acrylic copolymer solution, and then a crosslinking agent is further added thereto to prepare a solution of a pressure-sensitive adhesive composition.

The pressure-sensitive adhesive solution thus obtained is applied onto one surface of a release liner to a predetermined thickness, thereby forming a pressure-sensitive adhesive solution layer thereon. Subsequently, while the pressure-sensitive adhesive solution layer is not as yet completely dried, the pressure-sensitive adhesive solution layer is stuck to one surface of a stretch fabric. Thereafter, the pressure-sensitive adhesive solution layer is dried to form a pressure-sensitive adhesive layer, followed by crosslinking the pressure-sensitive adhesive layer, thereby producing a pressure-sensitive adhesive tape or sheet for application to skin of the present invention. Preferably, the stretch fabric to which the undried pressure-sensitive adhesive solution layer is stuck is previously subjected to a water-repellent treatment with a water repellent. In other words, in case where the undried pressure-sensitive adhesive solution layer is stuck to the stretch fabric which is not previously subjected to a water-repellent treatment, the pressure-sensitive adhesive may penetrate through the inside of the stretch fabric to the back thereof (back-through-penctration). However, when the stretch fabric is previously subjected to a water-repellent treatment, the pressure-sensitive adhesive solution layer hardly penetrate through the stretch fabric, so that the trouble of back-through-penetration can be avoided. In addition, as compared with a case of attaching a dried pressure-sensitive adhesive layer to the stretch fabric, the embodiment of attaching the undried pressure-sensitive adhesive layer to the stretch fabric is exhibits an excellent effect and is more advantageous in that the pressure-sensitive adhesive solution can readily penetrate into the inside of the stretch fabric (but to a degree not bringing about the trouble of back-through-penetration), and therefore the anchoring force of the pressure-sensitive adhesive layer to the stretch fabric is enhanced and the pressure-sensitive adhesive layer does not peel at the interface thereof to the stretch fabric even after repeated stretching operation of the pressure-sensitive adhesive tape.

### Examples

The present invention is described more specifically with reference to Examples given below. "Part" and "%" given below are all by weight.

### Example 1:

As a stretch fabric, prepared was a stretch smooth knit fabric of polyester 75-denier yarn, having a thickness of 450 µm and stretchable by 75% in the 75% in the cross direction and by 50% in the machine direction. As measured according to JIS-L1096, the physical data of the fabric were as follows: 20% modulus, 1.2 N/19 mm width; elongation, 115%; tensile strength, 93 N/19 mm width; elastic memory level, 82%.

This was dipped in an emulsion of an acrylic water repellent containing perfluoroalkyl (meth)acrylate as the main ingredient thereof (manufactured by Asahi Glass Co. Ltd.; trade name, Asahi Guard AG-8025), then wringed with a pinch roll and dried for water-repellent treatment.

On the other hand, as a pressure-sensitive adhesive layer, a monomer mixture containing 65 parts of isononyl acrylate, 30 parts of 2-methoxyethyl acrylate and 5 parts of acrylic acid was uniformly dissolved in mixed in 80 parts of toluene, and 0.3 parts of a polymerization initiator, azobisisobutyronitrile was added thereto, and the monomer mixture was copolymerized. Specifically, the monomer mixture was polymerized at about 60°C for about 10 hours, then heated up to 78°C, and further ripened for 2 hours. The gel fraction of the thus-obtained acrylic copolymer was 0.1%, the weight-average molecular weight thereof was 740,000, and the molecular weight distribution was 13.1.

Next, caprylic triglyceride was added to the toluene solution of the thus-produced acrylic acid copolymer in an amount of 45 parts relative to 100 parts of the acrylic copolymer therein, and an isocyanate-based crosslinking agent (manufactured by Nippon Polyurethane Industry Co., Ltd.; trade name, Coronate HL) was thereto in an amount of 0.075 parts. One surface of a release liner was processed with silicone; and the pressure-sensitive adhesive solution was applied onto the release-processed surface of the release liner to a dry thickness of 60 µm, thereby forming a pressure-sensitive adhesive solution layer thereon.

Next, while the pressure-sensitive adhesive solution layer was kept undried, this was stuck to one surface of the above-mentioned stretch fabric subjected to a water-repellent treatment, and dried at 150°C for 3 minutes to crosslink the pressure-sensitive adhesive layer, thereby producing a pressure-sensitive adhesive tape for application to skin of the present invention.

### Example 2:

Polymethane stretch yarn (11%, trade name, Lycra, manufactured by Du Pont-Toray Co., Ltd., 75 deniers) and polyester 75-denier processed yarn were knitted in a polyester spun-bond non-woven fabric (trade name, Ecule 6201A, TOYOBO CO., LTD., unit weight 20 g/m²) to prepare a stretch knit fabric controlled to be stretchable by 190% in the cross direction and by 90% in the machine direction. The physical data of the fabric were as follows: 20% modulus, 1.1 N/19 mm width; elongation, 108%; tensile strength, 91 N/19 mm width; elastic memory level, 86%.

The stretch fabric was subjected to a water-repellent treatment in the same manner as in Example 1. Using this, a pressure-sensitive adhesive tape for application to skin of the present invention was prepared in the same manner as in Example 1.

### Reference Example 3:

A pressure-sensitive adhesive tape for application to skin was prepared in the same manner as in Example 1, for which, however, the stretch fabric was not subjected to a water-repellent treatment.

### Example 4:

A pressure-sensitive adhesive layer was formed in the same manner as in Example 1, for which, however, the acrylic copolymer was prepared by uniformly mixing and dissolving 70 parts of 2-ethylhexyl acrylate, 25 parts of 2-ethoxyethyl acrylate and 5 parts of acrylic acid in 45 parts of toluene, and copolymerizing them therein. The gel fraction of the produced copolymer was 0%, the weight-average molecular weight thereof was 640,000, and the molecular weight distribution thereof was 11.4.

### Example 5:

A pressure-sensitive adhesive tape for application to skin of the present invention was prepared in the same manner as in Example 1, for which, however, triglyceryl 2-ethylhexanoate was used as the long-chain fatty acid glycerin ester in place of caprylic triglyceride.

### Comparative Example 1:

A pressure-sensitive adhesive tape for application to skin of the present invention was prepared in the same manner as in Example 1, for which, however, the long-chain fatty acid glycerin ester, caprylic triglyceride was not incorporated in the pressure-sensitive adhesive layer.

### Comparative Example 2:

A pressure-sensitive adhesive tape for application to skin of the present invention was prepared in the same manner as in Example 1, for which, however, a non-stretch, thick hard-twist fabric having a thickness of 730 µm was used in place of the stretch fabric. The physical data of the fabric were as follows: 20% modulus, 4 N/19 mm width; elongation, 110%; tensile strength, 196 N/19 mm width; elastic memory level, 83%.

### Comparative Example 3:

A pressure-sensitive adhesive tape for application to skin of the present invention was prepared in the same manner as in Example 1, in which, however, isopropyl myristate was incorporated in place of the long-chain fatty acid glycerin ester, caprylic triglyceride.

The pressure-sensitive adhesive tapes for application to skin prepared in the above-mentioned Examples and Comparative Examples were tested and evaluated in the manner mentioned below, and the results are shown in Table 1.

### <Adhesiveness to Skin in Taping>

The pressure-sensitive adhesive tapes prepared in Examples, Reference Example 3 and Comparative Examples were cut into strips having a width of 50 mm and a length of 400 mm. The strip was wound around the ankle joint of a human subject. After 24 hours, the condition of the tested adhesive tape was visually checked for the presence or absence of peeling or loosening, and evaluated according to the criteria mentioned below. The number of samples (n) is n = 10 human subjects.
A: No peeling occurred in 9 or more subjects (at least 90% of the samples).
B: No peeling occurred in from 5 to 8 subjects (from 50% to less than 90%), and the tape partly peeled or loosened in 2 to 5 subjects.
C: Peeling occurred in 6 or more subjects.

### <Skin Irritation>

The pressure-sensitive adhesive tapes prepared in Examples and Comparative Examples were cut into strips having a width of 50 mm and a length of 200 mm. The strip was attached to the anterior region of the forearm of a human subject under elongation to a suitable degree. After 8 hours in daily life, the pressure-sensitive adhesive tape was peeled away and evaluated based on the irritation that the subject had felt in its peeling. The criteria for the evaluation are as follows:
A: The subject felt a slight pain, or felt some irritation with little pain.
B: The subject felt a pain, or felt some irritation with a pain.

### <Adhesive Residue>

After the above-mentioned test for adhesiveness to skin, the tape was peeled away, and the skin surface was visually checked for the presence or absence of adhesive residue thereon. The tape was evaluated according to the criteria mentioned below.
A: The adhesive residue was less than 10% of the area of the pressure-sensitive adhesive tape attached to the skin.
B: The adhesive residue was from 10% to less than 50% of the area of the pressure-sensitive adhesive tape attached to the skin.
C: The adhesive residue was 50% or more of the area of the pressure-sensitive adhesive tape attached to the skin.

### <Waterproof Adhesiveness>

The tape was tested in the same manner as that for the above-mentioned test for the pressure-sensitive adhesiveness to skin in taping. During the test period of 24 hours, the subjects took a bath. After the bathing, the tape was tested and evaluated in the same manner as that for the above-mentioned test for the adhesiveness to skin.

**Table 1**

| Example or Comparative Example | Adhesiveness to Skin | Skin Irritation | Adhesive Residue | Waterproof Adhesiveness |
|---|---|---|---|---|
| Ex. 1 | A | A | A | A |
| Ex. 2 | A | A | A | A |
| Ref. Ex. 3 | A | A | A | B |
| Ex. 4 | A | A | A | A |
| Ex. 5 | A | A | A | A |
| Comp. Ex. 1 | A | B | A | A |
| Comp. Ex. 2 | B | B | A | A |
| Comp. Ex. 3 | B | A | B | B |

As is obvious from the results in Table 1 above, the pressure-sensitive adhesive tape or sheet for application to skin of the present invention is excellent in the adhesiveness to skin, especially in the adhesiveness to skin in taping applications, and causes little skin irritation, and its still another advantage is that, when peeled away, it does not leave die pressure-sensitive adhesive on the skin surface.

In addition, since the pressure-sensitive adhesive tape or sheet of the present invention includes a stretch fabric, it is elongable and has a tensile strength in some degree, and it has excellent clastic memory characteristics. Further, a specific pressure-sensitive adhesive is used in the tape or sheet, and therefore the pressure-sensitive adhesive layer has a suitable degree of moisture permeability. Accordingly, the presure-sensitive adhesive tape or sheet of the present invention has good adhesiveness to skin and exhibits another advantage in that it gives quite low irritation to the surface of skin. In particular, it is obvious that, when a fabric subjected to a water-repellent treatment is used, the pressure-sensitive adhesive tape or sheet exhibits excellent waterproofness in bathing.

Accordingly, the pressure-sensitive adhesive tape or sheet of the present invention has many applications in various fields of medicine, sports, or the like. In particular, the tape or sheet is especially suitable to taping applications, and its practicability is great.

## Claims

1. A pressure-sensitive adhesive tape or sheet for application to skin, which comprises:
a stretch fabric being a knitted fabric, and
a pressure-sensitive adhesive layer provided on one surface of the stretch fabric;
wherein the pressure-sensitive adhesive layer comprises:
an acrylic copolymer obtained from a monomer mixture comprising an alkyl (meth)acrylate of from 40 to 80% by weight, an alkoxy (meth)acrylate of from 10 to 50% by weight and a (meth)acrylic acid of from 1 to 10% by weight,
a long-chain fatty acid glycerin ester in an amount of from 20 to 100 parts by weight relative to 100 parts by weight of the acrylic copolymer, and
a crosslinking agent,
said pressure-sensitive adhesive layer being crosslinked with the crosslinking agent,
wherein a water-repellant film is not laminated on the fabric, and
wherein the stretch fabric is subjected to a water-repellent treatment with a water repellent.

2. The pressure-sensitive adhesive tape or sheet according to claim 1, wherein the water repellent is an emulsion containing an acrylic water repellent containing a perfluoroalkyl (meth)acrylate as a main ingredient thereof.

3. The pressure-sensitive adhesive tape or sheet according to claim 1, wherein the water-repellent treatment comprises dipping the stretch fabric in an emulsion containing the water repellent, wringing and drying the stretch fabric to thereby make an acrylic water repellent contained in the water repellent adhere to surfaces of fibers constituting the stretch fabric.

4. The pressure-sensitive adhesive tape or sheet according to claim 1, wherein a fraction of the acrylic copolymer soluble in a solvent for molecular weight determination has a weight-average molecular weight of from 500,000 to 1,000,000 and has a molecular weight distribution of from 8 to 15, wherein the weight-average molecular weight and the molecular weight distribution are data determined through gel permeation chromatography, the sample to be analyzed is dissolved in tetrahydrofuran, then the soluble fraction thereof having passed through a membrane filter having a diameter of 0.45 µm is analyzed, and the data are computed in terms of polystyrene.

5. The pressure-sensitive adhesive tape or sheet according to claim 1, wherein the acrylic copolymer has a molecular weight distribution of from 3 to 22.

6. The pressure-sensitive adhesive tape or sheet according to claim 1, wherein the stretch fabric has a degree of elongation of at least 30% in both the machine direction and the cross direction thereof.

7. A process for producing a pressure-sensitive adhesive tape or sheet, said process comprising:
applying a pressure-sensitive adhesive solution comprising an acrylic copolymer obtained from a monomer mixture comprising an alkyl (meth)acrylate of from 40 to 80% by weight, an alkoxy (meth)acrylate of from 10 to 50% by weight and a (meth)acrylic acid of from 1 to 10% by weight, a long-chain fatty acid glycerin ester in an amount of from 20 to 100 parts by weight relative to 100 parts by weight of the acrylic copolymer and a crosslinking agent, onto one surface of a release liner to a predetermined thickness to thereby form a pressure-sensitive adhesive solution layer thereon;
then attaching the pressure-sensitive adhesive solution layer on one surface of a stretch fabric being a knitted fabric while the pressure-sensitive adhesive solution layer is not completely dried; and
then drying the pressure-sensitive adhesive solution layer to form a pressure-sensitive adhesive layer, followed by crosslinking the pressure-sensitive adhesive layer,
wherein the stretch fabric is previously subjected to a water-repellent treatment with a water repellent.

8. The process according to claim 7, wherein the water repellent is an emulsion containing an acrylic water repellent containing a perfluoroalkyl (meth)acrylate as a main ingredient thereof.

9. The process according to claim 7, wherein the water-repellent treatment comprises dipping the stretch fabric in an emulsion containing the water repellent, wringing and drying the stretch fabric to thereby make an acrylic water repellent contained in the water repellent adhere to surfaces of fibers constituting the stretch fabric.

10. The process according to claim 7, wherein the stretch fabric has a degree of elongation of at least 30% in both the machine direction and the cross direction thereof.

## Patentansprüche

1. Haftklebeband oder -folie zur Anwendung auf die Haut, das bzw. die umfasst:
ein Stretch-Gewebe, welches eine Maschenware ist, und
eine Haftklebeschicht, die auf einer Oberfläche des Stretch-Gewebes bereitgestellt ist;
wobei die Haftklebeschicht umfasst:
ein Acrylcopolymer, erhalten aus einer Monomermischung umfassend ein Alkyl(meth)acrylat in einer Menge von 40 bis 80 Gew.-%, ein Alkoxy(meth)acrylat in einer Menge von 10 bis 50 Gew.-% und eine (Meth)acrylsäure in einer Menge von 1 bis 10 Gew.-%,
einen Glycerinester von einer langkettigen Fettsäure in einer Menge von 20 bis 100 Gewichtsteilen, bezogen auf 100 Gewichtsteile des Acrylcopolymers, und
ein Vernetzungsmittel,
wobei die Haftklebeschicht mit dem Vernetzungsmittel vernetzt ist,
wobei ein wasserabweisender Film nicht auf das Gewebe laminiert ist, und
wobei das Stretch-Gewebe einer Hydrophobierungsbehandlung mit einem Hydrophobiermittel unterzogen ist.

2. Haftklebeband oder -folie nach Anspruch 1, wobei das Hydrophobiermittel eine Emulsion ist, die ein Hydrophobiermittel auf Acrylbasis enthält, das ein Perfluoralkyl(meth)acrylat als einen Hauptbestandteil davon enthält.

3. Haftklebeband oder -folie nach Anspruch 1, wobei die Hydrophobierungsbehandlung das Eintauchen des Stretch-Gewebes in eine Emulsion, die das Hydrophobiermittel enthält, das Auswringen bzw. Auspressen und das Trocknen des Stretch-Gewebes umfasst, um dadurch ein in dem Hydrophobiermittel enthaltenes Hydrophobiermittel auf Acrylbasis an Oberflächen von Fasern anhaften zu lassen, die das Stretch-Gewebe bilden.

4. Haftklebeband oder -folie nach Anspruch 1, wobei eine Fraktion des Acrylcopolymers, die in einem Lösungsmittel zur Molekulargewichtsbestimmung löslich ist, ein gewichtsgemitteltes Molekulargewicht von 500000 bis 1000000 aufweist und eine Molekulargewichtsverteilung von 8 bis 15 aufweist, wobei das gewichtsgemittelte Molekulargewicht und die Molekulargewichtsverteilung durch Gelpermeationschromatographie bestimmte Daten sind, die zu analysierende Probe in Tetrahydrofuran gelöst wird, anschließend ihre lösliche Fraktion, die durch einen Membranfilter mit einem Durchmesser von 0,45 µm hindurchgegangen ist, analysiert wird und die Daten bezogen auf Polystyrol berechnet werden.

5. Haftklebeband oder -folie nach Anspruch 1, wobei das Acrylcopolymer eine Molekulargewichtsverteilung von 3 bis 22 aufweist.

6. Haftklebeband oder -folie nach Anspruch 1, wobei das Stretch-Gewebe einen Dehnungsgrad von wenigstens 30 % sowohl in seiner Maschinenrichtung als auch in seiner Querrichtung aufweist.

7. Verfahren zum Herstellen eines Haftklebebandes oder einer Haftklebefolie, wobei das Verfahren umfasst:
das Aufbringen einer Haftklebstofflösung umfassend ein Acrylcopolymer, erhalten aus einer Monomermischung umfassend ein Alkyl(meth)acrylat in einer Menge von 40 bis 80 Gew.-%, ein Alkoxy(meth)acrylat in einer Menge von 10 bis 50 Gew.-% und eine (Meth)acrylsäure in einer Menge von 1 bis 10 Gew.-%, einen Glycerinester von einer langkettigen Fettsäure in einer Menge von 20 bis 100 Gewichtsteilen, bezogen auf 100 Gewichtsteile des Acrylcopolymers, und ein Vernetzungsmittel, auf eine Oberfläche einer Trennlage bis zu einer vorbestimmten Dicke, um dadurch eine Haftklebstofflösungsschicht darauf zu bilden;
anschließend das Anbringen der Haftklebstofflösungsschicht an einer Oberfläche eines Stretch-Gewebes, welches eine Maschenware ist, während die Haftklebstofflösungsschicht nicht vollständig getrocknet ist; und
anschließend das Trocknen der Haftklebstofflösungsschicht, um eine Haftklebeschicht zu bilden, gefolgt vom Vernetzen der Haftklebeschicht,
wobei das Stretch-Gewebe zuvor einer Hydrophobierungsbehandlung mit einem Hydrophobiermittel unterzogen wird.

8. Verfahren nach Anspruch 7, wobei das Hydrophobiermittel eine Emulsion ist, die ein Hydrophobiermittel auf Acrylbasis enthält, das ein Perfluoralkyl(meth)acrylat als einen Hauptbestandteil davon enthält.

9. Verfahren nach Anspruch 7, wobei die Hydrophobierungsbehandlung das Eintauchen des Stretch-Gewebes in eine Emulsion, die das Hydrophobiermittel enthält, das Auswringen bzw. Auspressen und das Trocknen des Stretch-Gewebes umfasst, um dadurch ein in dem Hydrophobiermittel enthaltenes Hydrophobiermittel auf Acrylbasis an Oberflächen von Fasern anhaften zu lassen, die das Stretch-Gewebe bilden.

10. Verfahren nach Anspruch 7, wobei das Stretch-Gewebe einen Dehnungsgrad von wenigstens 30 % sowohl in seiner Maschinenrichtung als auch in seiner Querrichtung aufweist.

## Revendications

1. Ruban ou feuille adhésif autocollant pour application sur la peau, qui comprend:
un tissu étirable, qui est un tissu tricoté, et une couche d'adhésif autocollant prévue sur une surface du tissu étirable;
dans lequel la couche d'adhésif autocollant comprend:
un copolymère acrylique obtenu à partir d'un mélange de monomères comprenant de 40 à 80% en poids d'un alkyl (méth)acrylate, de 10 à 50% en poids d'un alcoxy (méth)acrylate et de 1 à 10% en poids d'un acide (méth)acrylique,
un ester d'acide gras à longue chaîne et de glycérol en quantité de 20 à 100 parties en poids pour 100 parties en poids du copolymère acrylique, et un agent de réticulation,
ladite couche d'adhésif autocollant étant réticulée par l'agent de réticulation,
dans lequel un film hydrofuge n'est pas lamifié sur le tissu, et
dans lequel le tissu étirable est soumis à un traitement hydrofuge à l'aide d'un agent hydrofuge.

2. Ruban ou feuille adhésif autocollant selon la revendication 1, dans lequel l'agent hydrofuge est une émulsion contenant un agent hydrofuge acrylique contenant un perfluoroalkyl(méth)acrylate en tant qu'ingrédient principal.

3. Ruban ou feuille adhésif autocollant selon la revendication 1, dans lequel le traitement hydrofuge comprend l'immersion du tissu étirable dans une émulsion contenant l'agent hydrofuge, l'essorage et le séchage du tissu étirable pour amener ainsi un agent hydrofuge acrylique contenu dans l'agent hydrofuge à adhérer aux surfaces des fibres constituant le tissu étirable.

4. Ruban ou feuille adhésif autocollant selon la revendication 1, dans lequel une fraction du copolymère acrylique soluble dans un solvant pour la détermination du poids moléculaire a un poids moléculaire moyen en poids de 500.000 à 1.000.000 et une distribution de poids moléculaire allant de 8 à 15, dans lequel le poids moléculaire moyen en poids et la distribution du poids moléculaire sont des données déterminées par chromatographie par perméation de gel, l'échantillon à analyser est dissous dans le tétrahydrofurane, puis sa fraction soluble, passée à travers un filtre à membrane présentant un diamètre de 0,45 µm est analysée, et les données sont calculées par rapport au polystyrène.

5. Ruban ou feuille adhésif autocollant selon la revendication 1, dans lequel le copolymère acrylique a une distribution du poids moléculaire de 3 à 22.

6. Ruban ou feuille adhésif autocollant selon la revendication 1, dans lequel le tissu étirable présente un degré d'allongement d'au moins 30% aussi bien dans le sens machine que dans le sens transversal.

7. Procédé de fabrication d'un ruban ou d'une feuille adhésif autocollant, ledit procédé comprenant les étapes consistant à :
appliquer une solution d'adhésif autocollant comprenant un copolymère acrylique obtenu à partir d'un mélange de monomères comprenant de 40 à 80% en poids d'un alkyl(méth)acrylate, de 10 à 50% en poids d'un alcoxy(méth)acrylate et de 1 à 10% en poids d'un acide (méth)acrylique, de 20 à 100 parties en poids d'un ester d'acide gras à longue chaîne et de glycérol, pour 100 parties en poids du copolymère acrylique, et un agent de réticulation sur une surface d'un revêtement anti-adhésif, sur une épaisseur prédéterminée, pour ainsi former sur celle-ci une couche de solution d'adhésif autocollant;
puis attacher la couche de solution d'adhésif autocollant à une surface d'un tissu étirable, qui est un tissu tricoté, alors que la couche de solution d'adhésif autocollant n'est pas complètement séchée ; et sécher ensuite la couche de solution d'adhésif autocollant pour former une couche d'adhésif autocollant, suivi d'une réticulation de la couche d'adhésif autocollant,
dans lequel le tissu étirable est d'abord soumis à un traitement hydrofuge avec un agent hydrofuge.

8. Procédé selon la revendication 7, dans lequel l'agent hydrofuge est une émulsion contenant un agent hydrofuge acrylique contenant un perfluoroalkyl(méth)acrylate en tant qu'ingrédient principal.

9. Procédé selon la revendication 7, dans lequel le traitement hydrofuge comprend l'immersion du tissu étirable dans une émulsion contenant l'agent hydrofuge, l'essorage et le séchage du tissu étirable pour amener ainsi un hydrofuge acrylique contenu dans l'agent hydrofuge à adhérer aux surfaces des fibres constituant le tissu étirable.

10. Procédé selon la revendication 7, dans lequel le tissu étirable présente un degré d'allongement d'au moins 30 % aussi bien dans le sens machine que dans le sens transversal.
